Europäisches Patentamt

European Patent Office

Office européen des brevets

(11) Publication number: **0 025 697**
**B1**

(12) **EUROPEAN PATENT SPECIFICATION**

(45) Date of publication of patent specification: **22.06.83**

(51) Int. Cl.³: **A 23 K 1/17, A 61 K 9/26**

(21) Application number: **80303199.6**

(22) Date of filing: **11.09.80**

(54) Growth promotant controlled release formulations and method of treatment.

(30) Priority: **12.09.79 US 74682**

(43) Date of publication of application:
**25.03.81 Bulletin 81/12**

(45) Publication of the grant of the patent:
**22.06.83 Bulletin 83/25**

(84) Designated Contracting States:
**DE GB LU NL SE**

(56) References cited:
**FR - A - 2 126 270**
**US - A - 3 773 919**
**US - A - 3 887 699**
**US - A - 3 937 836**
**US - A - 4 011 312**

(73) Proprietor: **ELI LILLY AND COMPANY**
**307, East McCarty Street**
**Indianapolis Indiana 46285 (US)**

(72) Inventor: **Kleber, John William**
**5335 Ashbourne Lane**
**Indianapolis Indiana (US)**
Inventor: **Simpson, Barbara Ellen**
**7401 East 71st Street**
**Indianapolis Indiana (US)**

(74) Representative: **Crowther, Terence Roger**
**European Patent Attorney et al,**
**Erl Wood Manor**
**Windlesham Surrey GU20 6PH (GB)**

Courier Press, Leamington Spa, England

# 0 025 697

## Growth promotant controlled release formulations and method of treatment

A number of agents are currently known which exhibit a beneficial effect upon feed consumption and utilization in animals when administered in conjunction with normal feed regimens. Some of the most important agents are those which increase the output by domestic animals of products utilized by humans. For example, ruminant animals, such as cattle, sheep and goats, experience an increase in the efficiency of their feed utilization as well as body growth promotion, and wool growth promotion in sheep, when administered effective amounts of growth promotors such as monensin, salinomycin, lasalocid and related compounds. Many of the growth promotors operate by altering the breakdown of food in the rumen of the animal. To be effective, such agents must be deposited in the rumen or reticulo-rumen of the animal.

A major problem associated with the drug administration to animals in general is the frequency required and the resulting rise in labor costs necessitated thereby. Moreover, many animals utilized for human food consumption, particularly ruminants such as cattle and sheep, are range fed for extended periods of time prior to feed lot development, thus rendering drug administration by daily dosing or by feed additives virtually impossible, and economically impracticable.

The administration of drugs to animals and humans by prolonged release formulations is known to be effective in some instances. For example, it is known to deposit a drug in an impregnable strip to be worn as a collar around the neck of an animal. The drug slowly releases for the prolonged treatment of external parasites. Reuter et al., in U.S.—A—4,011,312, discloses a prolonged release drug dosage form useful for the treatment of bovine mastitis. Such formulation is comprised of a suitable antimicrobial agent dispersed in a copolymer made up of about 60 to 80 mole percent glycolic acid and 20 to 40 mole percent lactic acid. Such copolymer is said to have a molecular weight of less than 2000. While the copolymer is effective in slowly releasing a therapeutic agent when in contact with the fluids of the teat canal, such copolymer is ineffective for beneficial controlled release when subjected to the fluids of the rumen.

The present invention provides formulations designed for controllably releasing an effective amount of growth promotant to a ruminant over a prolonged period of time. Also the invention provides a method for increasing feed utilization in ruminants. Such method is particularly important in the treatment of range fed animals, since a single treatment according to this invention is effective for several months.

The invention concerns a controlled release formulation capable of uniformly delivering an efficacious dose of a polyether antibiotic to a ruminant animal over a prolonged period of time following a single administration. The invention provides a formulation matrix that is biodegradable into substances naturally occuring in biological systems, with no undesirable residues remaining in animal tissues. More particularly, the invention concerns a biodegradable controlled release formulation useful in promoting growth and feed utilization in ruminant animals which comprises from 20 to 80 weight percent of a polyether antibiotic intimately dispersed throughout a copolymer derived from 60 to 95 weight percent of lactic acid and 40 to 5 weight percent glycolic acid, said copolymer having an inherent viscosity of 0.08 to 0.30 when measured in chloroform, said copolymer having a weight average molecular weight of 6000 to 35000.

A preferred copolymeric matrix utilized in the formulations of the invention is one derived from 60 to 90 weight percent lactic acid and 40 to 10 weight percent glycolic acid, and has an inherent viscosity of 0.10 to 0.25. A more preferred formulation is one wherein the copolymer contains from 70 to 80 weight percent lactic units and 30 to 20 weight percent glycolic units, with an inherent viscosity of 0.13 to 0.23 and a weight average molecular weight of 15000 to 30000.

This invention provides controlled release formulations useful for promoting the growth of ruminant animals and for enhancing utilization of feed by ruminants. The formulations contain as active ingredient from 20 to 80 weight percent of any of the well known and commonly utilized ruminant growth promoting agents which are polyether antibiotics such as monensin, narasin, lasalocid, salinomycin, alborixin, lysocellin, deshydroxymethyl monensin, nigericin, deshydroxymethyl nigericin, dianemycin, ionomycin, noboritomycin, deoxynarasin, and related growth promotors.

A particularly preferred formulation provided by this invention comprises a biodegradable dosage form useful for effecting growth promotion in ruminants comprising from 20 to 80 weight percent, most preferably 40 to 50 weight percent, of monensin or monensin sodium intimately dispersed throughout a copolymeric matrix derived from 60 to 90 weight percent of lactic acid and 40 to 10 weight percent of glycolic acid, said copolymer having an inherent viscosity of 0.10 to 0.25. Most preferably, the copolymer has 80 weight percent of lactic acid and 20 weight percent of glycolic acid and an inherent viscosity of 0.18 to 0.20.

This invention additionally provides a method for increasing the efficiency of feed utilization by a ruminant animal for a prolonged period of time which comprises orally administering to a ruminant an effective amount of a controlled release formulation made up of 20 to 80 weight percent of a polyether antibiotic intimately dispersed throughout a copolymer derived from 60 to 95 weight percent of lactic acid and 40 to 5 weight percent glycolic acid, said copolymer having an inherent viscosity of 0.08 to

2

0.30 when measured in chloroform, said copolymer having a weight average molecular weight of 6000 to 35000, said formulation being in combination with suitable excipients and carriers therefor, in such a way that the formulation is retained in the rumen or reticulo-rumen portion of the ruminant stomach. According to the method of this invention, ruminants can receive a continuous effective dose of growth promotant over a prolonged period of time following a single administration.

A preferred method according to the invention comprises orally administering to a ruminant an effective amount of a formulation comprised of 30 to 70 weight percent of monensin sodium salt admixed with 70 to 30 weight percent of a copolymer derived from 60 to 90 weight percent of lactic acid and 40 to 10 weight percent glycolic acid, with an inherent viscosity of 0.10 to 0.25.

The present invention provides a controlled release formulation which can be placed in the reticulo-rumen of a ruminant and which will uniformly deliver an efficacious dose of polyether antibiotic to the animal via the rumen for a period of 80 to 160 days. The formulations of this invention utilize a copolymeric material ideally suited to the controlled release of an effective amount of a pharmaceutical agent to an animal such that the animal can be effectively treated with a minimum of administrations. Such copolymeric material is prepared by a process which permits the substantially complete removal of polymerization catalyst, thereby permitting the total degradation of the copolymeric matrix in a biological system without the concomitant accumulation of toxic residues in animal tissues. This aspect of the invention is of particular significance in the treatment of animals utilized in the production of meat and other animal products intended for human consumption.

The copolymers required for the formulations of this invention are prepared by condensation of lactic acid and glycolic acid in the presence of a readily removable polymerization catalyst. Such catalysts include strong acid ion-exchange resins in the form of beads or similarly hard structures which are easily removed by filtration or similar techniques. Particularly preferred polymerization catalysts include commercially available strong acid ion-exchange resins such as Amberlite IR—118(H), Dowex HCR—W (formerly Dowex 50W), Duolite C—20, Amberlyst 15, Dowex MSC—1, Duolite C—25D, Duolite ES—26 and related strong acid ion-exchange resins. "Amberlite", "Dowex", "Amberlyst" and "Duolite" are registered Trade Marks. The catalyst is added to a mixture of 60 to 95 weight percent of lactic acid and 40 to 5 weight percent of glycolic acid. The amount of catalyst utilized is not critical to the polymerization, but typically is from 0.01 to 20.0 parts by weight relative to the total weight of combined lactic acid and glycolic acid. The polymerization generally is carried out in the absence of solvents; however, organic solvents such as dimethylsulfoxide or N,N-dimethylformamide can be utilized if desired. The polymerization reaction routinely is carried out in a reaction system equipped with a condensing system, thereby permitting the collection and removal of water that is formed, as well as facilitating the removal of any lactide and glyoclide by-products that are formed. The polymerization reaction generally is conducted at an elevated temperature of 100 to 250°C., and at such temperature is usually substantially complete within 48 to 96 hours. Ideally, the reaction can be carried out under a reduced pressure, thereby further facilitating removal of water and by-products.

The copolymer thus formed is readily recovered by simply filtering the molten reaction mixture, for example through a wire screen, to remove substantially all of the strong acid ion-exchange polymerization catalyst. Alternatively, the reaction mixture can be cooled to room temperature and then dissolved in a suitable organic solvent such as dichloromethane or acetone and then filtered by normal means so as to remove the solvent-insoluble strong acid ion-exchange resin. The copolymer then is isolated by removal of the solvent from the filtrate, for instance by evaporation under reduced pressure. Further purification of the copolymer can be accomplished if desired by re-dissolving it in a suitable organic solvent and further filtration, including the use of standard filter aids if desired.

The copolymer thus formed is required in the formulations and method of treatment provided by this invention. Such copolymers, while not amenable to exact structure elucidation, are characterized as having a weight average molecular weight of 6000 to 35000, and ideally 25000. The copolymers are unique in that they are classified as high molecular weight substances having an inherent viscosity from 0.08 to 0.30 when measured by standard techniques utilizing the Ubbelohde viscometer in which chloroform has an efflux time of about 51 seconds at 25°C. The inherent viscosity of the copolymers is determined by the following equations:

$$\eta r = t/t_o \qquad \eta inh = \frac{\ln \eta r}{C}$$

wherein:
$\eta r$ is relative viscosity;
$t_o$ is efflux time of solvent;
$t$ is efflux time of the solution;
$\eta inh$ is inherent viscosity;
$C$ is concentration in grams per 100 ml. of solvent; and
ln is logarithum.

The copolymers utilized in the formulations of this invention are additionally unique in that they are capable of providing a controlled release of pharmaceutical agents heretofore unavailable in ruminant fluids.

The formulations comprehended by this invention comprise an effective amount of a polyether antibiotic uniformly admixed and dispersed throughout the copolymeric matrix hereinabove described. The formulations contain 20 to 80 weight percent of active ingredient, ideally 30 to 70 weight percent. The pharmacologically active agents which can be utilized in the formulations include those agents commonly employed in the promotion of growth and stimulation of feed utilization by ruminants. Commonly used polyether antibiotics are monensin, narasin, lasalocid, salinomycin, alborixin, lysocellin, deshydroxymethyl monensin, nigericin, deshydroxymethyl nigericin, dianemycin, ionomycin, noboritomycin, deoxynarasin and others. It will be recognized that salts and esters of such compounds can also be used. A particularly preferred formulation according to this invention comprises the polyether antibiotic monensin (see U.S.—A—3,839,557) in the form of a sodium salt. Such growth promotant is preferably admixed with a copolymer containing 70 to 80 weight percent lactic acid and 30 to 20 weight percent glycolic acid, said copolymer having an inherent viscosity of 0.13 to 0.23. The formulations of the invention can, if desired, contain more than one active ingredient, as well as any of a number of commonly utilized pharmaceutical diluents, excipients and carriers.

The formulations provided by this invention can be prepared in any of a number of ways including dry mixing, and spray drying. A preferred method of preparation comprises dissolving a suitable amount of the aforementioned copolymer in a solubilizing organic solvent that is readily removed by evaporation, and then adding the desired amount of pharmacologically active agent, followed by removal of the organic solvent. For example, 50 grams of a copolymer derived from 80 weight percent of lactic acid and 20 weight percent of glycolic acid, having an inherent viscosity of 0.18, can be dissolved in 200 to 400 ml. of a suitable organic solvent such as dichloromethane, acetone, dimethyl ether, tetrahydrofuran, or chloroform. A pharmacologically active growth promoting agent, such as monensin sodium, lasalocid or salinomycin, in the amount of 40 to 50 g., is then added to the dissolved copolymer. The solution thus formed is stirred for uniform mixing and then the solvent is removed by evaporation, thus providing a uniformly mixed formulation of copolymer and active agent in a solid mass. The solid so formed can be placed in a suitable capsule for convenient oral administration to a ruminant. For instance, the formulation can be administered orally to a range fed calf for effective growth promotion and/or enhanced feed utilization over a prolonged period of time. Such treatment provides uniformly controlled release of growth promotion to the ruminant, such that the effective dose of active ingredient is safe for the animal. Said effective dose typically amounts to less than 500 mg. per animal each day. Typical daily doses will be 100 to 300 mg per animal. The novel formulation affords treatment to the animal for as long as 160 days.

The formulations of the invention can alternatively be prepared by first mixing the suitable copolymer and active agent in the powdered dry state in order to provide a uniform powdered mixture The mixture is next heated to 80 to 100°C. for six to ten hours to provide a uniformly mixed granulated formulation. The formulation so formed is next extruded, for instance, through a standard Killion Extruder, thereby providing a softened uniform mass which can be filled directly into a capsule.

The formulations provided by this invention can contain, in addition to the copolymer matrix and the active ingredient, other substances commonly utilized in medicinal formulations. Diluents, carriers, binders, excipients and adjuvants routinely incorporated in such formulations include gum tragacanth, acacia, corn starch, gelatin, alginic acid, magnesium stearate, aluminum monostearate, Span 80, Tween 80, sorbitan monostearate, hexaglyceryldistearate, glyceryldistearate, sucrose, lactose, methylparaben, propylparaben, bees wax, mannitol, propylene glycol, microcrystalline cellulose, calcium silicate, silica, polyvinylpyrrolidone, cocoa butter, polyoxyethylene sorbitan monolaurate, ethyl lactate, sorbitan, trioleate, calcium stearate, and talc, "SPAN" and "TWEEN" are registered Trade Marks.

The formulations contemplated herein can, if desired, include more than one pharmacologically active ingredient. Certain polyether antibiotics, for example, have an immediate onset of action, while others may not be completely effective until normal treatment has been carried out repeatedly. According to this invention, a fast acting polyether antibiotic can be combined with the aforementioned copolymer matrix, together with a slower acting active agent. Administration of such formulation is then effective for increasing feed utilization and promoting growth in the host animal for several months after a single administration. A particularly preferred formulation containing more than one active polyether antibiotic is one containing monensin and lasalocid.

The formulations of this invention are useful when administered to a ruminant and retained in the reticulo-rumen portion of the stomach of such ruminant. In order to function as contemplated, the formulation should be administered in a holder or capsule capable of being retained in the reticulo-rumen of the animal, and having sufficient exposed surface area such that the rumen fluids contact the formulation so that the desired controlled release of active agent is achieved. Any of a number of capsules designed to be retained in the rumen or reticulo-rumen portion of an animal can be utilized for the delivery of a formulation provided by this invention. Typical devices for administration of therapeutic and biologically active substances to ruminants are described in detail by Laby, U.S.—A— 3,844,285. These devices suffer, however, from having less than ideal exposed surface area. A particularly preferred receptacle for use in administering the formulations of this invention is a metal

4

cylinder open at both ends. More particularly, a steel cylinder measuring 20 to 35 mm. in diameter and 20 to 80 mm. in length, weighing from 60 to 120 grams when empty, is ideally suited to administration to a ruminant. Such steel capsule is of sufficient weight to remain in the reticulo-rumen of the ruminant. Such steel capsule can be made of mild steel or the like and, if desired, plated with a suitable metal such as nickel. Ideally, such steel cylinder is equipped with suitably spaced inner grooves 30 to 50 percent of the thickness of the cylinder wall. Such inner grooves will aid retention of the formulation while the device is lodged in the reticulo-rumen of the animal. Pins and similar cross bars can be provided as further retaining means if desired.

For administration to ruminants such as bovine, the steel bolus hereinabove described can be packed with 35 to 60 g. of a controlled release formulation of the invention. Such bolus then is ready for oral administration to a calf for the uniform release of the growth promotor and/or feed efficiency enhancer over a period of time of 80 to 160 days.

For ruminants such as sheep, a steel capsule 10 mm. to 20 mm. in diameter and 20 to 30 mm. in length, open at both ends, can be packed with a formulation of this invention for the controlled release of the desired growth promoting agent. Such agents, for instance monensin, cause an increase in feed utilization and/or effect growth in sheep not only of actual weight, but also of their generation of fleece.

The controlled release of active agent from the formulations according to this invention has been demonstrated in both *in vitro* and *in vivo* experiments. In a typical *in vitro* study, a steel capsule measuring 25 mm. by 25 mm. was packed with 11.0 grams of a fifty percent by weight formulation of monensin in a copolymer matrix derived from 80 weight percent lactic acid and 20 weight percent glycolic acid, said copolymer having an inherent viscosity of 0.18. The bolus thus prepared (total weight 39.25 g) was placed in a plastic bottle containing 200 ml. of artificial rumen fluid at pH 7.0, prepared according to the method of Cheng et al., *Journal of Dairy Science, 38,* 1225 (1955). The plastic bottle also contained twelve stainless steel ball bearings, each measuring 9 mm. in diameter. The bottom was rotated continuously at 34 rpm at a constant temperature of 39°C. Such conditions simulate the movement and abrasive effects of feed in the rumen of an animal. At twenty-four hour intervals over an eleven day test period, the capsule was removed, dried and weighed. The aqueous solution was removed from the plastic bottle at each twenty-four hour interval and assayed for its monensin content by the colorimetric method of Golab et al., *Journal A.O.A.C., 56,* 171 (1973). Fresh solution was placed in the bottle at each twenty-four hour interval.

The results of such *in vitro* experiment are given in Tables I and II below. Table I gives the daily weight reduction of the monensin bolus. Table II gives the quantity of monensin found each day in the rumen-like fluid.

TABLE I

Monensin *in vitro* capsule weight change

| days | 0 | 1 | 2 | 3 | 4 | 5 | 6 | 7 | 8 | 9 | 10 | 11 |
|---|---|---|---|---|---|---|---|---|---|---|---|---|
| Weight (grams) | 39.25 | 38.75 | 38.2 | 37.7 | 37.2 | 36.7 | 36.25 | 35.8 | 35.45 | 34.8 | 34.3 | 33.75 |
| Weight change per day (grams) | | —0.5 | —0.55 | —0.5 | —0.5 | —0.5 | —0.45 | —0.45 | —0.35 | —0.65 | —0.5 | —0.55 |
| Cumulative weight change (grams) | | —0.5 | —1.05 | —1.55 | —2.05 | —2.55 | —3.0 | —3.45 | —3.8 | —4.45 | —4.95 | —5.5 |

0025697

## TABLE II

Daily Monensin content of *in vitro* rumen fluid

| Day | 1 | 2 | 3 | 4 | 5 | 6 | 7 | 8 | 9 | 10 | 11 |
|---|---|---|---|---|---|---|---|---|---|---|---|
| mg. found | 215 | 228 | 231 | 210 | 208 | 181 | 247 | 151 | 270 | 257 | 178 |
| mg. theory | 250 | 275 | 250 | 250 | 250 | 225 | 225 | 175 | 325 | 250 | 275 |
| Percent of theory found | 86 | 83 | 92 | 84 | 83 | 80 | 110 | 86 | 83 | 103 | 65 |

The *in vitro* data presented in Tables I and II demonstrate that in a simulated rumen environment, a controlled release formulation of this invention is effective in delivering a controlled and substantially uniform daily dose of active ingredient over an extended period of time.

The controlled release formulations provided by this invention additionally have been evaluated in *in vivo* systems. In one such study, mature cattle were equipped with a fistula for ready access to the reticulo-rumen portion of the stomach. Preweighed steel boluses, containing a formulation consisting of 50 weight percent of monensin sodium salt and 50 weight percent of a copolymer derived from 80 weight percent lactic acid and 20 weight percent glycolic acid, were placed, via the fistula, into the rumen of each of three heifers. The animals were permitted to graze as desired, and were allowed to drink water freely. The formulation filled bolus was removed from the animals, via the fistula, at 7 to 13 day intervals over about a three month test period. Each bolus was weighed to determine the amount of active ingredient which had been administered to each animal, and then each bolus was returned to the reticulo-rumen via the fistula. The payout of active ingredient to each of the three test animals is given in Table III.

TABLE III

Payout of Monensin from formulations placed in the rumen of fistulated cattle

|  | Animal #1 | Animal #2 | Animal #3 |
|---|---|---|---|
| Gross bolus weight | 142.8 g | 142.3 g | 143.3 g |
| Empty bolus weight | 98.8 g | 97.9 g | 99.0 g |
| Net formulation weight (50% monensin) | 44.0 g | 44.4 g | 44.3 g |

| Payout periods (days) | Estimated Monensin payout mg/head/day (weight loss of the bolus divided by 2) | | |
|---|---|---|---|
| 0—7 | 0 | 96 | 100 |
| 7—14 | 164 | — | 221 |
| 14—21 | 243 | 193 | 150 |
| 21—29 | 194 | 200 | 206 |
| 29—42 | 227 | 192 | 246 |
| 42—52 | 175 | 185 | 170 |
| 52—63 | 182 | 195 | 227 |
| 63—72 | — | 183 | 239 |
| 73—79 | — | 71 | 100 |
| 79—86 | — | 121 | 150 |

According to the data presented in Table III, the average daily payout of monensin, from a formulation of this invention containing fifty percent by weight of monensin sodium salt, is about 169 mg/head/day for animal #1, 160 mg/head/day for animal #2 and 181 mg/head/day for animal #3, or a mean average daily dose of about 170 mg/head. Such uniform dosing is continuous for about three months. Once all of the formulation contained in the steel capsule has been released, the empty capsule is of such weight that it simply remains in the reticulo-rumen. Additional filled capsules can be administered as needed, and all such capsules can be removed at the time of slaughter. Such removed capsules can be cleaned and repacked with the same or a different formulation and re-administered to ruminant animals, thereby adding economical benefits to the present invention.

A further embodiment of this invention is a method for promoting the growth of ruminant animals and increasing the efficiency of feed utilization by ruminants over a prolonged period of time. Such method of treatment comprises orally administering to a ruminant a growth promoting amount of a controlled release formulation comprised of a growth promoting agent admixed with a copolymer derived from 60 to 95 weight percent of lactic acid and 40 to 5 weight percent of glycolic acid and having an inherent viscosity of 0.08 to 0.30. The method provided herein comprises administering a single dose of a formulation as described hereinabove, which administration is effective for controllably delivering to the reticulo-rumen of a ruminant an efficacious daily dose of the active growth promoting agent over a prolonged period of time from 80 to 160 days. Additional single doses of such controlled release formulations can be administered so as to obtain the desired treatment over an indefinite period of time with a minimum number of such administrations.

The method provided by this invention is ideally suited to the growth promotion of ruminants which are not amenable to receiving food additives. Such method renders the growth promotion of range fed ruminants particularly attractive and economical. The method provided by this invention comprehends administering a controlled release formulation containing an effective amount of growth promotor such that the daily dose of active agent to an animal is safe, yet effective to stimulate feed utilization and/or promote growth when the animal partakes of the available food source. The method will be practiced by orally administering an effective amount of controlled release formulation to a ruminant weighing, in the case of sheep, 18.2—90.9 kg (40—200 lbs), and for bovine, 90.9—909 kg (200—2000 lbs). The method is preferably directed to cattle weighing 90.9—727 kg (200—1600 lbs). Typical range fed calves treated according to this invention will weigh 159—273 kg (350—600 lbs).

The daily dose of active ingredient delivered to a host animal according to this invention will be from 25 to 1000 mg., and such daily dose preferably will be from 25 to 500 mg. per head. It will of course be recognized that the daily dose provided to a host animal will vary somewhat depending upon the concentration of active ingredient utilized in the controlled release formulation, in addition to the particular growth promoting agent incorporated in the formulation and the specific copolymeric matrix utilized. A preferred method according to the invention comprises administering a controlled release formulation of a growth promotor such as monensin, salinomycin or lasalocid, such that the typical dosage is, for sheep, 5 to 20 mg/head/day for an animal weighing 18.2—68.2 kg (40—150 lbs), and for calves, 50 to 200 mg/head/day, for animals weighing 182—364 kg (400—800 lbs). It is also preferred that such method be carried out utilizing a formulation capable of delivering the desired daily dosage uniformly over a period of time of 80 to 120 days. The method according to this invention includes repeated administration so as to obtain the desired treatment for the desired extended period of time.

The method of this invention can be practiced on any ruminant animal, including cattle utilized for beef production, cows utilized for milk production or herd proliferation, sheep which are utilized for meat production or for wool production, and for goats utilized for meat, and milk.

In an effort to more fully illustrate particular aspects of this invention, the following detailed examples of the preparation of copolymers and final formulations of the invention are provided. The examples are representative only and should not be construed as limiting in any respect.

## Example 1

To a 3-neck round bottom flask equipped with a condenser and thermometer were added 864.0 g. of lactic acid, 201.0 g. of glycolic acid and 12.0 g. of Dowex HCR—W2—H ion exchange resin. The mixture was stirred and heated to 130°C. for three hours, during which time 400 ml. of water were distilled and collected. After discarding the water thus produced, stirring and heating were continued and the pressure was gradually reduced by vacuum over three hours, after which time the temperature of the reaction mixture had increased to 150°C. at a final pressure of 670 Nm$^{-2}$ (5 torr). An additional 12.0 g. of Dowex HCR—W2—H catalyst was added to the reaction mixture, and the mixture then was heated to 170°C. at 670 Nm$^{-2}$ (5 torr) for twenty-four hours, and then at 185°C. at 670 Nm$^{-2}$ (5 torr) for an additional 48 hours. The molten reaction mixture next was filtered to remove most of the ion exchange polymerization catalyst, and the filtrate was allowed to cool to room temperature to give 700 g. of 80 percent lactic — 20 percent glycolic copolymer. The copolymer was analyzed by proton nuclear magnetic resonance spectrometry and shown to be comprised of 76 percent by weight of lactic units.

The viscosity of the copolymer was determined in a Ubbelohde viscometer in which chloroform had an efflux time of 51 seconds at 25°C. The copolymer was dissolved in chloroform at a concentration of 0.50 g. per 100 ml. of solvent. Inherent viscosity of the copolymer was then determined according to the formulas:

$$\eta\text{rel} = \frac{t}{t_o}$$

$$\eta\text{inh} = \frac{\ln \eta\text{rel}}{C}$$

wherein:

$\eta$rel = relative viscosity
$t_o$ = efflux time of solvent ($CHCl_3$)
$t$ = efflux time of solution
$\eta$inh = inherent viscosity
$C$ = conc. in grams/100 ml.

The inherent viscosity of the copolymer thus prepared was determined to be 0.19 dl/g.

### Example 2

Following the general procedure set forth in Example 1, 432 g. of lactic acid and 101 g. of glycolic acid were condensed in the presence of a total of 12.0 g. of Amberlyst 15 ion exchange polymerization catalyst to afford 350 g. of a copolymer comprised of about 80 percent lactic units and about 20 percent glycolic units. The copolymer had the following inherent viscosity: 0.18 dl/g.

### Example 3

Following the general procedure of Example 1, 422.0 g. of lactic acid were condensed with 144.0 g. of glycolic acid in the presence of a total of 12.0 g. of Dowex HCR—W2—H ion exchange polymerization catalyst. After removing the catalyst by filtration of the molten reaction mixture, there was provided 350 g. of a copolymer derived from 75 percent by weight of lactic acid and 25 percent by weight of glycolic acid. The copolymer exhibited the following inherent viscosity: 0.19 dl/g.

### Example 4

Following the general procedure of Example 1, 1080 g. of lactic acid were condensed with 252 g. of glycolic acid in the presence of a total of 30.0 g. of Dowex HCR—W2—H ion exchange polymerization catalyst to give, after removal of the catalyst, 750 g. of a copolymer which was shown by proton NMR to contain 79 percent of lactic units and 21 percent of glycolic units. The copolymer exhibited the following inherent viscosity: 0.20 dl/g.

### Example 5

Following the procedure of Example 1, 1080 g. of lactic acid were condensed with 120 g. of glycolic acid in the presence of a total of 15.0 g of Dowex HCR—W2—H ion exchange polymerization catalyst to provide, after work-up, 630 g. of a copolymer derived from about 90 weight percent of lactic acid and about 10 weight percent of glycolic acid. The copolymer had an inherent viscosity of 0.20 dl/g.

### Example 6

Following the procedure of Example 1, 710 g. of lactic acid were condensed with 190 g. of glycolic acid in the presence of a total of 12.0 g. of Dowex HCR—W2—H ion exchange polymerization catalyst to provide 500 g. of a copolymer comprised of about 70 percent lactic units and about 30 percent glycolic units. The copolymer had an inherent viscosity of: 0.12 after 24 hours at 175°C.

### Example 7

The procedure of Example 1 was followed to condense 1080 g. of lactic acid with 120 g. of glycolic acid in the presence of a total of 30.0 g. of Dowex HCR—W2—H ion exchange polymerization catalyst. After workup, there was recovered 750 g. of a copolymer derived of about 89 weight percent of lactic acid and about 11 weight percent of glycolic acid having an inherent viscosity of 0.20 dl/g.

The copolymers provided by this invention additionally have been characterized by gel permeation chromatography (high pressure liquid chromatography) and subsequent determination of molecular weight. Gel permeation chromatography separates sample molecules by differences in effective molecular size in solution. Separation is accomplished as a result of the pore size distribution in the packing material. This analytical technique allows determinations of weight-average molecular weight, number average molecular weight, molecular weight distribution, and dispersity for polymeric materials.

Several such experiments have been carried out on the copolymers of this invention. Standard gel permeation chromatographic columns were used, and the support in each case was commercial $\mu$ Styragel. All samples and standards were dissolved in a solution of 80 parts tetrahydrofuran and 20 parts dichloromethane. The indirect method (i.e. the "Q-Factor Method") of calibrating the gel permeation chromatographic columns was used to obtain molecular weight averages for the copolymers of the invention. Commercial polystyrene, with a Q Factor of 41.3, was used in the calibrations. The following Table presents several determinations of molecular weight by standard gel permeation chromatographic techniques as outlined above. A more detailed discussion of the technique utilized is presented by Slade in *Polymer Molecular Weights,* Marcel Deckker, Inc., 1975.

In the Table, column I presents the relative proportions of lactic units and glycolic units making up the copolymer analyzed. Column II gives the inherent viscosity of each copolymer analyzed. Column III reports the strong acid ion exchange resin utilized to prepare the copolymer being analyzed. Column IV presents the weight average angstrom size as determined from the gel permeation chromatographic retention time for the particular copolymer. Column V presents the weight average molecular weights for the various copolymers prepared by the process of this invention. The weight average molecular weights are determined by multiplying the Q-Factor for polystyrene (41.3) times the weight average angstrom size for the particular copolymer being analyzed. Column VI is the relevant Example number.

As demonstrated in the Table, the preferred copolymers of this invention have a molecular weight from 15,000 to 35,000, and ideally from 15,000 to 30,000.

### Table of Weight Average Molecular Weights

| Column I | II | III | IV | V | VI |
|---|---|---|---|---|---|
| 80:20 | 0.19 | Dowex HCR—W2—H | 432.5 | 17,862 | 1 |
| 80:20 | 0.18 | Amberlyst 15 | 412.3 | 17,027 | 2 |
| 75:25 | 0.19 | Dowex HCR—W2—H | 505.9 | 20,894 | 3 |
| 80:20 | 0.20 | Dowex HCR—W2—H | 777.1 | 32,094 | 4 |
| 90:10 | 0.20 | Dowex HCR—W2—H | 749.3 | 30,946 | 5 |
| 70:30 | 0.12 | Dowex HCR—W2—H | 400.5 | 16,541 | 6 |
| 90:10 | 0.20 | Dowex HCR—W2—H | 522.1 | 21,563 | 7 |

### Example 8

To a stirred solution of 150 ml. of dichloromethane containing 22.0 g. of a copolymer derived from 80 weight percent of lactic acid and 20 weight percent of glycolic acid, having an inherent viscosity of 0.19, was added in one portion 22.0 g. of monensin sodium salt. The solution was stirred at ambient temperature for ten minutes and then the solvent was removed by evaporation under reduced pressure. The solid mass that was obtained was ground and heated to 100°C. and packed into a steel capsule measuring 35 mm × 50 mm and weighing 98.8 g. Final weight of the packed capsule was 142.8 g.

### Example 9

To a stirred solution of 200 ml. of chloroform containing 40.0 g. of the copolymer derived from about 90 weight percent lactic acid and 10 weight percent glycolic acid, having an inherent viscosity of 0.20, was added 50.0 g. of salinomycin, 40 g. of beeswax and 500 mg. of sorbitan monostearate. The solution was stirred at 25°C. for several minutes and then the solvent was removed by evaporation under reduced pressure. The residue was dissolved in fresh chloroform and spray dried by conventional means to provide a controlled release formulation of salinomycin ideally suited for oral administration in the form of a molded pill that is weighted with sufficient iron filings.

### Example 10

A formulation comprised of 5.0 g. of monensin sodium salt in 7.0 g. of a copolymer derived from 60 weight percent lactic acid and 40 weight percent glycolic acid, having an inherent viscosity of 0.20, was extruded into rods and then melted at 100°C. and packed into a steel cylinder measuring 10 mm in diameter and 20 mm in length, said steel cylinder being open at both ends. The bolus then prepared was orally administered to a sheep weighing 31.8 kg (70 lbs) for the effective promotion of growth over a four month period.

## Claims

1. A biodegradable controlled release formulation useful in promoting growth and feed utilization in ruminant animals which comprises from 20 to 80 weight percent of a polyether antibiotic intimately dispersed throughout a copolymer derived from 60 to 95 weight percent of lactic acid and 40 to 5 weight percent of glycolic acid, said copolymer having an inherent viscosity of 0.08 to 0.30 when measured in chloroform, said copolymer having a weight average molecular weight of 6000 to 35000.

2. The formulation of claim 1 wherein the active polyether antibiotic is monensin, narasin, lasalocid, salinomycin, alborixin, lysocellin, deshydroxymethyl monensin, nigericin, deshydroxymethyl nigericin, dianemycin, ionomycin, noboritomycin, or deoxynarasin, and the pharmaceutically acceptable salts and esters thereof where appropriate.

3. The formulation of claim 2 wherein the active polyether antibiotic is monensin, monensin sodium, lasalocid, narasin, salinomycin, and nigericin.

4. The formulation of claim 3 wherein the active polyether antibiotic is monensin sodium.

5. The formulation of claim 1 wherein the active polyether antibiotic is present in 30 to 70 percent by weight.

6. The formulation of claim 5 wherein monensin sodium is present in 40 to 50 percent by weight.

7. The formulation of claim 1 wherein the copolymer is derived from 60 to 90 weight percent lactic acid and 40 to 10 weight percent glycolic acid and has an inherent viscosity of 0.10 to 0.25.

8. The formulation of claim 7 wherein the copolymer is derived from 70 to 80 weight percent of lactic acid and 30 to 20 weight percent glycolic acid.

9. The formulation of claim 8 wherein the copolymer is derived from 70 to 80 weight percent of lactic acid and 30 to 20 weight percent glycolic acid, and has an inherent viscosity of 0.13 to 0.23.

10. The formulation of claim 7 wherein the copolymer is derived from 80 weight percent lactic acid and 20 weight percent glycolic acid.

11. The formulation of claim 1 when packed or compressed into a steel open ended cylinder measuring 20 to 35 millimeters in diameter and 20 to 80 millimeters in length, containing 35 to 60 grams of formulation.

12. A method for increasing the efficiency of feed utilization by a ruminant animal for a prolonged period of time which comprises orally administering to the ruminant an effective amount of the controlled release formulation of claim 1 in such a way that the formulation is retained in the rumen or reticulo-rumen portion of the ruminant stomach.

13. The method of claim 12 wherein the formulation administered is any one of claims 1 to 11.

## Patentansprüche

1. Bioabbaubare Formulierung mit gesteuerter Wirkstoff-freigabe, die sich zur Verbesserung des Wachstums und der Futterverwertung bei Wiederkäuern eignet, dadurch gekennzeichnet, daß diese Formulierung aus 20 bis 80 Gewichtsprozent eines Polyetherantibiotikums besteht, das innig dispergiert ist in einem Copolymerisat aus 60 bis 95 Gewichtsprozent Milchsäure und 40 bis 5 Gewichtsprozent Glykolsäure, wobei dieses Copolymerisat in Chloroform gemessen eine grundmolare Viskosität vor 0,08 bis 0,30 hat und ein gewichtsmittleres Molekulargewicht von 6000 bis 35000 aufweist.

2. Formulierung nach Anspruch 1, dadurch gekennzeichnet, daß sie als wirksames Polyetherantibiotikum Monensin, Narasin, Lasalocid, Salinomycin, Alborixin, Lysocellin, Deshydroxymethylmonensin, Nigericin, Deshydroxymethylnigericin, Dianemycin, Ionomycin, Noboritomycin oder Desoxynarasin oder geeignetenfalls die pharmazeutisch unbedenklichen Salze und Ester hiervon enthält.

3. Formulierung nach Anspruch 2, dadurch gekennzeichnet, daß sie als wirksames Polyetherantibiotikum Monensin, Natriummonensin, Lasalocid, Narasin, Salinomycin oder Nigericin enthält.

4. Formulierung nach Anspruch 3, dadurch gekennzeichnet, daß sie als wirksames Polyetherantibiotikum Natriummonensin enthält.

5. Formulierung nach Anspruch 1, dadurch gekennzeichnet, daß sie das wirksame Polyetherantibiotikum in einer Menge von 30 bis 70 Gewichtsprozent enthält.

6. Formulierung nach Anspruch 5, dadurch gekennzeichnet, daß sie Natriummonensin in einer Menge von 40 bis 50 Gewichtsprozent enthält.

7. Formulierung nach Anspruch 1, dadurch gekennzeichnet, daß das Copolymerisat aus 60 bis 90 Gewichtsprozent Milchsäure und 40 bis 10 Gewichtsprozent Glykolsäure besteht und eine grundmolare Viskosität von 0,10 bis 0,25 hat.

8. Formulierung nach Anspruch 7, dadurch gekennzeichnet, daß das Copolymerisat aus 70 bis 80 Gewichtsprozent Milchsäure und 30 bis 20 Gewichtsprozent Glykolsäure besteht.

9. Formulierung nach Anspruch 8, dadurch gekennzeichnet, daß das Copolymerisat aus 70 bis 80 Gewichtsprozent Milchsäure und 30 bis 20 Gewichtsprozent Glykolsäure besteht und eine grundmolare Viskosität von 0,13 bis 0,23 hat.

10. Formulierung nach Anspruch 7, dadurch gekennzeichnet, daß das Copolymerisat aus 80 Gewichtsprozent Milchsäure und 20 Gewichtsprozent Glykolsäure besteht.

11. Formulierung nach Anspruch 1, dadurch gekennzeichnet, daß sie in einen offenendigen Stahlzylinder mit einem Durchmesser von 20 bis 35 mm und einer Länge von 20 bis 80 mm gepackt oder komprimiert ist, der 35 bis 60 g Formulierung enthält.

12. Verfahren zur Verbesserung der Futterverwertung eines Wiederkäuers über eine verlängerte Zeitdauer, dadurch gekennzeichnet, daß man dem Wiederkäuer oral eine wirksame Menge der Formulierung mit gesteuerter Wirkstoffabgabe von Anspruch 1 in einer Weise verabreicht, daß die For-

**0 025 697**

mulierung im Rumen oder Reticulorumen des Magens des Wiederkäuers verbleibt.

13. Verfahren nach Anspruch 12, dadurch gekennzeichnet, daß eine Formulierung nach einem der Ansprüche 1 bis 11 verabreicht wird.

**Revendications**

1. Formulation biodégradable à libération contrôlée, utile pour favoriser la croissance et l'utilisation des aliments chez les ruminants, caractérisée en ce qu'elle contient 20 à 80% en poids d'un antibiotique de polyéther dispersé intimement dans un copolymère dérivant de 60 à 95% en poids d'acide lactique et de 40 à 5% en poids d'acide glycolique, ce copolymère ayant une viscosité inhérente de 0,08 à 0,30, mesurée dans le chloroforme, ainsi qu'un poids moléculaire moyen en poids de 6.000 à 35.000.

2. Formulation suivant la revendication 1, caractérisée en ce que l'antibiotique de polyéther actif est la monensine, la narasine, le lasalocide, la salinomycine, l'alborixine, la lysocelline, la deshydroxyméthyl-monensine, la nigéricine, la deshydroxyméthyl-nigéricine, la dianémycine, l'ionomycine, la noboritomycine ou la déoxynarasine, ainsi que leurs sels et esters pharmaceutiquement acceptables lorsqu'ils sont appropriés.

3. Formulation suivant la revendication 2, caractérisée en ce que l'antibiotique de polyéther actif est la monensine, la monensine sodique, le lasalocide, la narasine, la salinomycine et la nigéricine.

4. Formulation suivant la revendication 3, caractérisée en ce que l'antibiotique de polyéther actif est la monensine sodique.

5. Formulation suivant la revendication 1, caractérisée en ce que l'antibiotique de polyéther actif est présent à raison de 30 à 70% en poids.

6. Formulation suivant la revendication 5, caractérisée en ce que la monensine sodique est présente à raison de 40 à 50% en poids.

7. Formulation suivant la revendication 1, caractérisée en ce que le copolymère dérive de 60 à 90% en poids d'acide lactique et de 40 à 10% en poids d'acide glycolique, tandis qu'il a une viscosité inhérente de 0,10 à 0,25.

8. Formulation suivant la revendication 7, caractérisée en ce que le copolymère dérive de 70 à 80% en poids d'acide lactique et de 30 à 20% en poids d'acide glycolique.

9. Formulation suivant la revendication 8, caractérisée en ce que le copolymère dérive de 70 à 80% en poids d'acide lactique et de 30 à 20% en poids d'acide glycolique, tandis qu'il a une viscosité inhérente de 0,13 à 0,23.

10. Formulation suivant la revendication 7, caractérisée en ce que le copolymère dérive de 80% en poids d'acide lactique et de 20% en poids d'acide glycolique.

11. Formulation suivant la revendication 1, cette formulation étant tassée ou comprimée dans un cylindre en acier à extrémités ouvertes ayant un diamètre de 20 à 35 mm et une longueur de 20 à 80 mm, ce cylindre contenant 35 à 60 g de la formulation.

12. Procédé en vue d'accroître l'efficacité d'utilisation des aliments par un ruminant au cours d'une période prolongée, caractérisé en ce qu'il consiste à administrer, par voie orale au ruminant, une quantité efficace de la formulation à libération contrôlée suivant la revendication 1 de telle sorte que cette formulation soit retenue dans le rumen ou le réticulo-rumen de l'estomac du ruminant.

13. Procédé suivant la revendication 12, caractérisé en ce que la formulation administrée est l'une quelconque des formulations des revendications 1 à 11.